# EUROPÄISCHE PATENTSCHRIFT

(11) **EP 2 124 972 B1**
(45) Veröffentlichungstag und Bekanntmachung des Hinweises auf die Patenterteilung: **13.06.2012**
(21) Anmeldenummer: 07857063.7
(22) Anmeldetag: 21.12.2007
(51) Int. Cl.: A61K 33/24, A61K 9/00, A61K 9/22, A61K 9/32, A61K 9/48, A61K 9/50, A61P 1/06, A61P 1/12, A61P 1/14, A61K 9/08

(54) **VERWENDUNG EINER ZUSAMMENSETZUNG AUS MINERALSTOFFEN UND GEGEBENENFALLS ACETOGENEN UND/ODER BUTYROGENEN BAKTERIEN ZUR VERMEIDUNG ODER REDUZIERUNG VON GASBILDUNG IM DICKDARM EINES SÄUGETIERS UND DADURCH BEDINGTER ABDOMINALER BESCHWERDEN**
USE OF A COMPOSITION MADE OF MINERAL NUTRIENTS AND OPTIONALLY ACETOGENIC AND/OR BUTYROGENIC BACTERIA IN ORDER TO AVOID OR REDUCE THE FORMATION OF GAS IN THE LARGE INTESTINE OF A MAMMAL AND THE RESULTING ABDOMINAL PROBLEMS
UTILISATION D'UNE COMPOSITION DE SUBSTANCES MINÉRALES ET ÉVENTUELLEMENT DE BACTÉRIES ACÉTOGÈNES ET/OU BUTYROGÈNES POUR EMPÊCHER OU DIMINUER LA FORMATION DE GAZ DANS LE GROS INTESTIN D'UN MAMMIFÈRE ET LES DOULEURS ABDOMINALES QUI EN RÉSULTENT

(30) Priorität: 22.12.2006 DE 102006062250
(43) Veröffentlichungstag der Anmeldung: 02.12.2009
(60) Teilanmeldung: 12163061.0
(73) Patentinhaber: Saur-Brosch, Roland, 69126 Heidelberg (DE)
(72) Erfinder: Saur-Brosch, Roland, 69126 Heidelberg (DE)
(74) Vertreter: Patentanwälte Bitterich, Dr. Keller, Schwertfeger
(86) Internationale Anmeldenummer: PCT/EP2007/011352
(87) Internationale Veröffentlichungsnummer: WO 2008/077614

(56) Entgegenhaltungen:
- EP-A- 0 585 208
- WO-A-00/10582
- WO-A-02/07741
- DE-A1- 10 003 096
- US-A- 5 997 911
- US-A1- 2004 005 304
- US-A1- 2004 253 320
- US-A1- 2005 207 956
- DATABASE WPI Week 200575 Thomson Scientific, London, GB; AN 2005-730947 XP002502896 & JP 2005 298417 A (JAPAN SCI & TECHNOLOGY AGENCY) 27. Oktober 2005 (2005-10-27)
- DATABASE WPI Week 198940 Thomson Scientific, London, GB; AN 1989-288840 XP002502897 & JP 01 211529 A (TANAKA T) 24. August 1989 (1989-08-24)
- DATABASE WPI Week 200351 Thomson Scientific, London, GB; AN 2003-536033 XP002502898 & JP 2003 040783 A (TANAKA H) 13. Februar 2003 (2003-02-13)
- DATABASE WPI Week 198515 Thomson Scientific, London, GB; AN 1985-089617 XP002502899 & JP 60 038326 A (OKUMURA C) 27. Februar 1985 (1985-02-27)
- "Metabolic data for tungsten" ANNALS OF THE ICRP, PERGAMON, Bd. 6, Nr. 2-3, 1. Januar 1981 (1981-01-01), Seiten 93-95, XP022919353 ISSN: 0146-6453 [gefunden am 1981-01-01]

## Beschreibung

Die vorliegende Erfindung betrifft eine Zusammensetzung, umfassend einen oder mehrere Mineralstoffe ausgewählt aus der Gruppe bestehend aus Selen, Molybdän oder Wolfram, die galenisch oder chemisch so ausgeführt ist, dass der Mineralstoff oder die Mineralstoffe vollständige oder teilweise erst kurz vor dem, beim oder kurz nach dem Erreichen des Dickdarmes freigesetzt werden, und deren Verwendung zur Herstellung eines Arzneimittels zur Verabreichung an ein Säugetier, für die Vermeidung oder Reduzierung von Gasbildung im Dickdarm und dadurch bedingter abdominaler Beschwerden, insbesondere Blähungen, Meteorismus oder Bauchkrämpfen.

Die Erfindung betrifft weiter ein Arzneimittel zur Vermeidung oder Reduzierung von Gasbildung im Dickdarm eines Säugetiers und dadurch bedingter abdominaler Beschwerden, bestehend aus einer Zusammensetzung, die einen oder mehrere Mineralstoffe ausgewählt aus der Gruppe bestehend aus Selen, Molybdän und Wolfram oder einer Kombination von diesen, und gegebenenfalls einen pharmazeutisch verträglichen Träger umfasst.

Ferner betrifft die Erfindung ein Verfahren zur Isolierung von acetogenen und butyrogenen Bakterienstämmen, die für die oben genannten therapeutischen Zwecke geeignet sind.

### Technisches Gebiet:

Die Erfindung liegt auf dem Gebiet der Medizin und Pharmakologie.

### Stand der Technik:

Eine große Anzahl von Menschen leidet an abdominalen Beschwerden, die größtenteils durch unerwünschte Stoffwechselprodukte von Mikroorganismen ausgelöst werden, die den menschlichen Darm besiedeln. Zu diesen Stoffwechselprodukten gehören insbesondere in dem Darm gebildete Gase (vornehmlich Gase wie Wasserstoff, Kohlendioxid, Methan, Schwefelwasserstoff und Stickstoff) sowie Milchsäure, kurzkettige Fettsäuren und verschiedene Enterotoxine. Je nach Art und Menge dieser Stoffwechselprodukte entstehen für den Betroffenen verschiedenartig ausgeprägte Beschwerden. Die gebildeten Gase führen beispielsweise zu abdominalen Beschwerden wie Blähungen, Meteorismus, Bauchkrämpfen und sonstigen darauf zurückgehenden Folgeerkrankungen. Zu solchen Folgeerkrankungen gehört beispielsweise eine bakterielle Überbesiedelung des Dünndarms (SIBO) in Folge einer durch übermäßigen dickdarmseitigen Gasdruck gestörten Funktion der Ileocaecalklappe. Bakteriell gebildete Milchsäure kann aufgrund ihrer osmotischen Wirkung zu Durchfällen führen und kann bei einer Absorption durch die Dickdarmschleimhaut pathologische Folgen haben. Wird von den Darmbakterien eine zu hohe Konzentration weniger effizient abbaubarer D-(-)-Milchsäure (d. h. linksdrehender Milchsäure) gebildet, kann es auch zu dem Krankheitsbild einer Lactatazidose kommen.

Enterotoxine können bei Aufnahme in den Blutkreislauf einen Einfluss auf das Nervensystem haben. Beispielsweise können sie Kopfschmerzen, Müdigkeit, Gereiztheit oder sogar einen Bewusstseinsverlust hervorrufen. Ferner können sie lähmend auf die Darmmuskulatur wirken und Obstipationen hervorrufen. Durch eine Reizung der Darmschleimhaut mit anschließender Freisetzung von Flüssigkeit können die Stoffwechselprodukte der Bakterien auch Durchfälle auslösen. Bakteriell gebildete Alkohole werden über die Dickdarmschleimhaut aufgenommen und belasten die Leber, die durch die andauernde Alkoholbelastung geschädigt werden kann.

Solche Beschwerden treten vermehrt und in höherer Intensität auf, wenn diese Stoffwechselprodukte in größeren Mengen anfallen, was vor allem dann der Fall ist, wenn die Bakterien, die diese Stoffwechselprodukte erzeugen, über normale Maße hinaus mit Nährstoffen versorgt werden. Dies ist unter anderem der Fall bei Kohlenhydratmalabsorptionen wie zum Beispiel durch Laktasemangel induzierter Lactosemalabsorption, Fructosemalabsorption, Malabsorptionen von Zuckeralkoholen (z. B. Sorbit, Xylit, Mannit, Lactit. Maltit), Disaccharasemangel und Trehalasemangel, Maldigestionen, die zurückgehen auf eine exokrine Pankreasinsuffizienz, Magensäuremangel, Gallensäuremangel oder ungenügender Einspeichelung des Nahrungsbreis, Zöliakie, Sprue, Gluten-Unverträglichkeit, bei Störungen des Kauapparates und ebenso bei vermehrter Aufnahme von unverdaulichen Nahrungsbestandteilen wie resistenter Stärke oder unverdaulichen Inhaltsstoffen aus Kohl und Hülsenfrüchten. Nach Dünndarmresektionen kommt es oft zu teilweisen Malabsorptionen/Maldigestionen aufgrund einer reduzierten Absorptionsfläche (Kurzdarmsyndrom).

Auch bei einer mengenmäßig normalen Gasproduktion kann es zu abdominalen Beschwerden kommen, wenn die Nerven im Darmtrakt überempfindlich reagieren. Dies ist beispielsweise dann der Fall, wenn wie bei einem Reizdarmsyndrom schon bei einer normalen Dehnung des Darmes, bei der ein normal gesunder Mensch lediglich eine vermehrte Darmtätigkeit registrieren würde, Schmerzen signalisiert werden. Bei Säuglingen treten infolge der noch nicht ganz ausgereiften Verdauungsorgane oft Malabsorptionen und Maldigestionen auf, die sich dann als sogenannte Dreimonatskoliken erkennbar machen.

Ferner kann es nach Antibiotikabehandlungen des Öfteren zum Auftreten von Darmbeschwerden kommen, was vermutlich auf die Veränderungen in der Darmflorazusammensetzung zurückzuführen ist (z.B. antibiotikainduzierte Dysbiose). Bestimmte antibiotikaresistente Bakterien, wie z. B. *Clostridium perfringens* und *Clostridium difficile* und Hefen wie z. B. *Candida albicans* vermehren sich aufgrund fehlender Nährstoffkonkurrenz übermäßig und tragen durch ihre vermehrt gebildeten Stoffwechselprodukte zu den Beschwerden bei.

Bei Maldigestionen basierend auf exokriner Pankreasinsuffizienz, Magensäuremangel oder Gallensäuremangel stehen üblicherweise gut wirksame Medikamente zur Verfügung. Allerdings kommt es in einigen Fällen trotzdem nicht zu einer vollständigen Eliminierung der Beschwerden, da sowohl die Dosierung als auch die Verteilung im Speisebrei (z. B. bei Substitution von Pankreasenzymen) nicht in der Weise vorgenommen werden können, wie bei einer ordnungsgemäßen Organfunktion.

Die Behandlung von Malabsorptionen besteht üblicherweise aus einer Diät, bei der auf die malabsorbierten Nährstoffe weitgehend bis vollständig verzichtet wird. Dies hat unter Umständen sehr starke Einschränkungen in der Wahl der Lebensmittel zur Folge und bringt oft auch keine vollständige Symptomfreiheit, da bestimmte Nährstoffe, wie z. B. Fructose in fast allen Lebensmitteln zumindest in Spuren vorhanden sind.

Bei der Lactosemalabsorption besteht ferner die Möglichkeit, das fehlende Enzym Laktase in Form von Pulvern, Tabletten oder Kapseln dem Körper zuzuführen, jedoch ergeben sich dabei ähnliche Unzulänglichkeiten hinsichtlich der Dosierung und der Verteilung im Speisebrei, wie bei der Substitution von Pankreasenzymen. Außerdem werden diese Enzyme teilweise aus Pilzkulturen extrahiert, so dass die damit hergestellten Produkte für manchen Allergiker nicht verträglich sind.

Eine weitere Möglichkeit zur Behandlung von Lactosemalabsorption besteht in der oralen Zufuhr von Lactose verstoffwechseinden Milchsäurebakterien (Lactobacillus spp. und Bifidobacterium spp.). Die Milchsäurebakterien verstoffwechseln dann einen gewissen Anteil der malabsorbierten Lactose zu Milchsäure, so dass eine geringere Konzentration von Lactose bis in den Dickdarm gelangt und dort somit weniger problematische Stoffwechselprodukte entstehen. Sofern diese Verstoffwechselung im Dünndarm erfolgt, kann ein Großteil der entstandenen Milchsäure resorbiert werden. Der nicht resorbierte Anteil und die erst im Dickdarm erzeugte Milchsäure kann jedoch weiterhin osmotisch wirksam sein und Durchfälle auslösen.

Da die Durchgangsdauer des Speisebreis durch den Dünndarm relativ kurz ist, kann während dieser Zeit auch nur eine begrenzte Menge Lactose verstoffwechselt werden, so dass mit dieser Methode nur bei recht geringen Lactosemengen eine nahezu vollständige Beschwerdefreiheit erzielt werden kann. Im Dickdarm treten die zugeführten Bakterien zwar auch in Nahrungskonkurrenz zu den Problemstoff bildenden Bakterien, es findet jedoch eher eine parallele Verstofwechselung statt, so dass immer noch erhebliche Mengen der problematischen Stoffwechselprodukte entstehen.

Die Verabreichung von milchsäurebildenden Bakterien (Lactobacillus spp. und Bifidobacterium spp.) wird auch bei anderen Störungen als der Lactosemalabsorption angewendet, mit dem Ziel. die Bakterien, welche die problematischen Stoffwechselprodukte erzeugen, zu verdrängen, um so eine Linderung herbeizuführen. Da sich bestimmte Bakterien nur bis zu einer bestimmten Maximalanzahl pro Menge Darminhalt vermehren können, weil dann entweder nicht genügend essentielle Nährstoffe zur Verfügung stehen oder eine Hemmung durch deren eigene Stoffwechselprodukte erfolgt, können problematische Bakterien durch solche Verdrängungsversuche nicht vollständig beseitigt werden. Außerdem haben Milchsäurebakterien oft nur ein recht begrenztes Spektrum an verstoffwechselbaren Substraten so dass bei der Verursachung der Symptome durch verschiedene unverdauliche Kohlenhydrate meist nicht alle die Symptome verursachenden Lebensmittel entschärft werden. Bei den sogenannten Dreimonatskoliken zum Beispiel besteht die Therapie vor allem in der Verabreichung von Entschäumem (z. B. Simethicon) und der Anwendung von Bauchmassagen, um die Blähungen zum schnelleren Verlassen des Darmes als Flatus zu bewegen. Auch die Verabreichung von Milchsäurebakterien wird empfohlen, wobei allerdings die gleichen Beschränkungen hinsichtlich der Wirksamkeit auftreten, wie bei der Behandlung der oben genannten abdominalen Störungen.

Teilweise wird auch versucht, die Beschwerden durch Verabreichung von Antibiotika zu lindern, mit dem Ziel die Bakterien, welche die beschwerdeauslösenden Stoffwechselprodukte produzieren, abzutöten. Das Problem bei diesem Vorgehen besteht jedoch darin, dass trotzdem unverdauliche Nährstoffe den Dickdarm erreichen und dort einen Wachstumsanreiz für Bakterien darstellen, die diese Stoffe verwerten können. In den meisten Fällen wird die Darmflora nach einer solchen Antibiotikatherapie sich selbst überlassen, in der öffnung, dass sich eine günstige Bakterienkonstellation von selbst einpendelt, was leider sehr oft nicht von dauerhaftem Erfolg gekrönt ist.

Einige Therapeuten verordnen Mittel zum Aufbau der Darmflora, die hauptsächlich Milchsäurebakterien oder nicht pathogene Enterobakterien (*Escherichia coli*) enthalten. Diese können den Darmfloraaufbau durch die Ansäuerung des Darmlumens und die Besetzung der Darmschleimhaut durchaus positiv beeinflussen, stellen aber keinen zielgerichteten Weg dar, eine unproblematische Entsorgung der durch die Bakterien gebildeten Gase sicherzustellen. Durch die Applikation der zuvor genannten Bakterien kann durch Nahrungskonkurrenz unter Umständen sogar eine zufällige Ansiedelung der erwünschten acetogenen Bakterien verhindert werden.

Auch bei Antibiotikatherapien, die nicht primär zur Bekämpfung bestimmter Bakterien der Darmflora verschrieben werden, versucht man durch Applikation von Müchsäurebakterien oder medizinischen Hefen einen positiven Wiederaufbau der Darmflora zu erreichen, die allerdings die oben beschriebenen Unzulänglichkeiten aufweisen.

Nollet L. et. al., Appl Microbiol Biotechnol, 1997, 48: 99 beschreiben die Wirkung der Zugabe von *Poptostreptococcus productus* in einem künstlichen Reaktorsystem, das den menschlichen Darm simuliert, auf die Darmflora und deren Aktivität. Die Autoren zeigen, dass die Methanproduktion durch Bakterien zu Gunsten der Acetogenese (d. h. die Umsetzung von Kohlenstoffdioxid (CO2) und Wasserstoff (H2) zu Essigsäure) durch Zugabe dieses Bakterienstammes verändert werden kann. Allerdings lässt diese Wirkung recht schnell nach, wenn die Verabreichung der Bakterien in das System beendet wird. Zwar produzieren die acetogenen Bakterien noch weiterhin Essigsäure, jedoch primär durch heterotrophe Homoacetogenese und nicht durch die erwünschte hydrogenotrophe reduktive Acetogenese. Daher wäre dieser Ansatz für eine pharmazeutische Zusammensetzung nicht zufriedenstellend.

Ein Bakterienstamm mit ähnlichen biochemischen Eigenschaften, *Ruminococcus hydrogenotrophicus,* wird von Bernalier A. et. al., Arch Microbiol, 1996, 166: 176 beschrieben. Der Stamm wurde aus menschlichen Fäkalien extrahiert und ist *in vitro* zur H2/CO2-Umsetzung fähig.

In den Patentanmeldungen US 2004/002 86 89 und WO 02/07741 werden nicht lebensfähige oder attenuierte pathogene Clostridien mit einem oder mehreren anderen nicht pathogenen oder attenuierten pathogenen Mikroorganismen zur Beeinflussung der Mikroflora im Darm verabreicht. Die Verabreichung von acetogene Bakterien mit oder ohne Mineralstoffe/Vitamine wird jedoch nicht erwähnt.

Ein anderer Ansatz wird in der DE 102 06 995 versucht. In dieser Druckschrift wird ein Präbiotika enthaltendes Mikronährstoffkombinationsprodukt beschrieben, das als diätisches Lebensmittel für medizinische Zwecke eingesetzt werden kann. Das Kombinationsprodukt enthält Bakterien wie Bifidobacterium, Lactobacillus, Enterococcus oder andere probiotische Kulturen zusammen mit Vitaminen und Mineralstoffen. Das Kombinationspräparat soll beispielsweise bei Allergien, Chemo- und Strahlentherapie, Gastroenteritiden, chronischentzündlichen Darmerkrankungen oder Lactoseintoleranz eingesetzt werden. Weitere relevante Dokumente sind WO00/0582 und US 2004 253320.

Aus der Tatsache, dass die meisten Menschen mit gastrointestinalen Störungen, die Malabsorptionen verursachen, keine oder nur geringfügige Beschwerden haben, und diese Menschen gegenüber symptombehafteten Malabsorbem erhöhte Mengen an Essigsäure im Stuhl aufweisen (Born et al.), kann davon ausgegangen werden, dass Säugetiere mit einer ausreichenden reduktiv acetogenen Stoffwechselleistung ihrer bakteriellen Darmflora keine oder nur unwesentliche Beschwerden durch Malabsorptionen haben, und dass bei Säugetieren, bei denen nach dem Verzehr von Lebensmitteln, die für sie nicht absorbierbare Nahrungsbestandteile enthalten, abdominale Beschwerden auftreten, die reduktiv acetogene Stoffwechselleistung der bakteriellen Darmflora vermindert ist.

Es ist daher Aufgabe der vorliegenden Erfindung eine Zusammensetzung und ein Verfahren bereitzustellen, die/das zur Vorbeugung oder Behandlung erhöhter Gasbildung im Dickdarm und dadurch bedingter abdominaler Beschwerden oder gastrolntestinaler Störungen geeignet ist.

Die Lösung der Aufgabe besteht in der Bereitstellung einer Zusammensetzung, die einen oder mehrere Mineralstoffe ausgewählt aus der Gruppe bestehend aus Selen, Molybdän und Wolfram oder einer Kombination von diesen, und gegebenenfalls einen pharmazeutisch verträglichen Träger umfasst. Diese Zusammensetzung ist zur Vorbeugung oder Behandlung erhöhter Gasbildung im Dickdarm und dadurch bedingter abdominaler Beschwerden wie beispielsweise Blähungen, Meteorismus, Bauchkrämpfen, Dreimonatskoliken, Irritated Bowel Syndrom, Reizdarm, oder zur Behandlung gastrointestinaler Störungen, welche Gasbildung im Dickdarm zur Folge haben können, verwendbar. Von der Erfindung umfasst sind ferner Arzneimittel oder Nahrungsergänzungsmittel, welche die zuvor genannten Mineralstoffe umfassen, und für die Reduktion oder Vermeidung von Gasbildung im Dickdarm verwendel werden.

Vorteilhafte Ausführungsformen der vorliegenden Erfindung ergeben sich aus den Unteransprüchen.

### Darstellung der Erfndung:

Die Erfindung basiert auf der überraschenden Entdeckung, dass Selen, Molybdän und insbesondere Wolfram, allein oder beliebiger Kombination, eine deutliche Reduzierung oder Vermeidung von Gasbildung im Dickdarm eines Säugetiers bewirken. Eine solche dickdarmgeziette Wirksamkeit der zuvor genannten Mineralstoffe zur Behandlung und Vorbeugung von durch eine erhöhte Gasbildung bedingten abdominalen Beschwerden *in vivo* ist neu und überraschend.

Der Begriff "Mineralstoff oder Mineralstoffe", wie hier verwendet, insbesondere die in der Erfindung beanspruchten Mineralstoffe Selen, Molybdän und Wolfram umfassen Naturstoffe, Reinstoffe, chemisch modifizierte Varianten davon, Verbindungen, Derivate und Analoge des jeweiligen Mineralstoffes. Beispiele hierfür sind Natriumwolframat, Natriumselenit, Natriumselenat, Selenomethionin, Natriummolybdat, Selenhefe, Wolframphytat, Selenphytal, Wolframoxalat, Wolframtannin.

Durch die erfindungsgemäße Mineralstoffzusammensetzung kann eine deutliche Reduzierung der Gasmenge im Darm erreicht werden, die bei den vorgenannten gastrointestinalen Störungen zu einer deutlichen Besserung der Beschwerden führt.

In bestimmten Fällen kann es notwendig sein, dass neben den beanspruchten Mineralstoffen ein oder mehrere acetogene oder butyrogene Bakterienstämme gleichzeitig, nacheinander oder als Bestandteil der Zusammensetzung dem Säugetier verabreicht werden. Durch die Wirkung der beanspruchten Mineralstoffe wird die Population und/oder Stoffwechselaktivität der Bakterien so gefördert, dass die Anzahl und/oder Menge der verabreichten Bakterien möglichst gering gehalten und/oder die Verabreichungsfrequenz gesenkt werden kann. Diese Variante ist Bestandteil einer bevorzugten Ausführungsform.

Ein Vorteil der Erfindung besteht darin, auch Beschwerden, die nicht hauptsächlich durch das vermehrte Aufkommen von Darmgasen verursacht werden, sondern von einer durch malabsorbierte Nahrungsbestandteile ausgelösten osmotisch bedingten vermehrten Flüssigkeitsmenge im Darm, behandeln zu können.
Dadurch können mehrere Hauptsymptome der genannten gastrointestinalen Störungen, weiche oft auch in Kombination auftreten, gleichzeitig durch eine einzige kombinierte Zusammensetzung behandelt werden.

Der Erfinder hat festgestellt, dass die Vermehrungsfähigkeit der für die Gasreduktion verantwortlichen acetogenen Bakterien im Darm eines Säugetiers (wie z. B. der Kolon eines Menschen) größtenteils davon abhängt, wie gut die Mikroorganismen mit Energiequellen (z. B. verwertbaren Kohlenhydraten), und Zellbaustoffen (z. B. Stickstoff) sowie mit weiteren Co-Faktoren (z. B. Vitamine und Mineralstoffen) versorgt werden. Insbesondere hat der Erfinder der voriiegenden Erfindung festgestellt, dass die gewünschte Stoffwechselaktivität der einzelnen Bakterien dagegen davon abhängt, in welchen Konzentrationen sie mit bestimmten Mineralstoffen versorgt werden, und mit welcher Aktivität die benötigten Enzyme für die jeweiligen Stoffwechselvorgänge somit gebildet werden können.

Auch bei ständiger erneuter Zufuhr acetogener Bakterien ergibt sich das Problem, dass die Bakterien sich zwar möglicherweise im Laufe der Darmpassage zahlenmäßig vermehren, sich die reduktiv acetogene Stoffwechselleistung der gesamten acetogenen Bakterien jedoch nicht wesentlich erhöht, da mangels benötigter Mineralstoffe die Gesamtmenge der Enzyme nicht signifikant erhöht werden kann.

Ferner ist eine ausreichende Versorgung der dickdarmständigen acetogenen Bakterien mit den benötigten Mineralstoffen über eine normalen Diät nicht möglich, da die benötigten Schwermetalle vermutlich bereits im Dünndarm zu einem großen Teil von der Darmschleimhaut absorbiert werden und so im Dickdarm nicht mehr in ausreichender Menge den acetogenen Bakterien zur Verfügung stehen.

Auch eine gezielte Erhöhung der entsprechenden Mineralstoffe in der Diät stellt keine Lösung des Problems dar, da diese in höheren Konzentrationen toxisch für den Wirtsorganismus sind. Hier greift nun die vorliegende Erfindung, in der die Nachteile der bekannten Verfahren und Bakterienstämme auf wirksame Weise überwunden werden. Sie beeinflusst zum einen die Anzahl acetogener Bakterien und das Gleichgewicht der Population von in der Darmflora bereits vorhandenen acetogenen Bakterien. Zum anderen fördert sie die zur Vermeidung von Gasbildung erforderlichen Stoffwechselwege acetogener Bakterien.

Die Erfindung schlägt zur Vermeidung abdominaler Beschwerden eine pharmazeutisch wirksame Zusammensetzung vor, welche die für die reduktive Acetogenese benötigten Schwermetalle Selen, Molybdän und/oder Wolfram, entweder allein oder in beliebiger Kombination enthält und vorzugsweise in einer galenischen Form zubereitet wird, so dass die wirksamen Bestandteile erst kurz vor, bei oder kurz nach dem Erreichen des Dickdarms freigesetzt werden. Dadurch wird die Verfügbarkeit der Schwermetallionen für die acetogenen Bakterien des Darms erheblich gesteigert und gleichzeitig der Anteil der vom Wirtsorganismus absorbierten Schwermetalle stark vermindert, so dass die Versorgung der acetogenen Bakterien sichergestellt wird, ohne den Wirtsorganismus einer zu hohen Schwermetalldosis auszusetzen.

Für Fälle, in denen die acetogenen Bakterien mit geringeren Mineralstoffkonzentrationen auskommen und diese mit Darreichungsformen erreicht werden, die nicht zur möglichst ausschließlichen Freisetzung im Dickdarm konzipiert sind, ohne den Wirt einer akuten Vergiftungsgefahr auszusetzen, schlägt die Erfindung außerdem eine Zusammensetzung vor, die nicht dünndarmresistent umhüllt ist.

Erfindungsgemäß haben sich die Mineralstoffe Selen, Wolfram und Molybdän zur Vermehrung und Förderung acetogener Bakterien und deren reduktiv acetogener Stoffwechselleistung im Dickdarm und damit zur Reduktion oder Vermeidung der Gasbildung als vorteilhaft erwiesen.

Die Mineralstoffe können einzeln oder in beliebiger Kombination in der Zusammensetzung entweder als Reinstoff, Naturstoff, chemische Verbindung, Derivat oder Analog enthalten sein. Beispielsweise können die Mineralstoffe so modifiziert sein, dass deren Freisetzung und Wirksamkeit im Dickdarm gefördert wird. Dies kann durch Bindung des Mineralstoffes an eine chemische Verbindung erreicht werden.

In einer bevorzugten Ausführungsform enthält die Erfindung zusätzlich Vanadium, Nickel, Eisen, Natrium, oder Kalium, entweder einzeln oder in beliebiger Kombination, auch mit den zuvor genannten Mineralstoffen.

Daneben haben sich die folgenden Vitamine für die Vermehrungsfähigkeit und Stoffwechselaktivität acetogener Bakterien als vorteilhaft erwiesen: Vitamin A, Vitamin B, Vitamin C, Vitamin D, Vitamin E, Vitamin K.

Bevorzugt sollen Mineralstoffe und Vitamine in einer Verabreichungsform in der Zusammensetzung enthalten sein. Je nach Zustand der Darmflora und Krankheitsbild sollte die Zusammensetzung zusätzlich einen oder mehrere Stämme acetogener oder gegebenenfalls butyrogener Bakterien und/oder Enterobakterien entfalten.

Die bevorzugte Verabreichungsform für die erfindungsgemäße Zusammensetzung und daraus hergestellte Arzneimittel oder Nahrungsergänzungsmittel ist eine orale oder rektale Verabreichung.

Die galenische Aufbereitung kann beispielsweise aus einer Magensaft- und Dünndarmresistenten Kapsel oder Tablette bestehen, deren Hülle sich nach einer bestimmten Zeit nach dem Verlassen des sauren Magenmilieus im Dickdarm auflöst.

Vorzugsweise wird die Zusammensetzung und ihre aktiven Bestandteile innerhalb eines Zeitraumes von 3 Stunden, vorzugsweise 6 Stunden, am meisten bevorzugt 12 Stunden im Dickdarm kontinuierlich freigesetzt. Diese Zeit soll das Intervall von einer Hauptmahlzeit zur anderen überbrücken. Bei täglicher Zunahme über mehrere Tage stellt sich die erwünschte Wirkung ein (siehe Beispiele 1 und 4).

Vorzugsweise besteht die galenische Aufbereitung aus einer Kapsel, deren Hülle durch Enzyme von dickdarmständigen Bakterien aufgelöst wird. Dem Fachmann stehen für die erfindungsgemäße galenische Aufbereitung die weiteren bekannten Techniken zur Verfügung, die in der vorliegenden Erfindung eingesetzt werden können. Durch die Verwendung einer galenischen Zubereitung und gezielte Freisetzung im Dickdarm, kann die Darmflora acetogener Bakterien gezielt aufgebaut oder regeneriert werden. Durch den Zusatz von Mineralstoffen und/oder Vitaminen ist ferner die langfristige Erhaltung der Population und Stoffwechselaktivität dieser Stämme und damit der Therapieerfolg sichergestellt, ohne dass eine laufende Verabreichung lebensfähiger acetogener Bakterien notwendig ist. Die Aufnahme von acetogenen Bakterien kann sich nämlich in einigen Fällen auch als nachteilig herausstellen, wenn beispielsweise der Körper gegen einen bestimmten (nicht autologen) Stamm empfindlich reagiert oder Allergien entwickelt Die Aufnahme von Mineralstoffen oder Vitaminen hingegen führt zur einer gezielten Stimulation von autologen Bakterien, die bereits in der Darmflora vorhanden sind. Auch bei Verwendung autologer Stämme von acetogenen Bakterien zur Erhöhung der Populationsdichte im Darm ist es vorteilhaft eine andauernde Einnahme vermeiden zu können, da Präparate mit autologen Stämmen naturgemäß aufwendig und teuer in der Herstellung sind, da sie für jedes Individuum separat hergestellt werden müssen, also (im Gegensatz zu einem Mineralstoffpräparat) nicht großtechnisch produziert werden können.

Bevorzugt sind die Mineralstoffe in einer Konzentration von 1 µg bis 3 mg pro Element und/oder die Vitamine in einer Konzentration von 1 IE bis 1000 IE (IE = Internationale Einheit) und/oder die acetogenen Bakterien in einer Konzentration von 1x105 bis 1x1013 KBE (KBE = Koloniebildende Einheit) und/oder die butyrogenen Bakterien in einer Konzentration von 1x105 bits 1x1013 KBE in der Zusammensetzung enthalten.

Eine weitere Ausgestaltung der Erfindung schlägt vor, den acetogenen Bakterien die benötigten Mineralstoffe als chemische Verbindungen zur Verfügung zu stellen, die im oder vom Darm des Wirtes nicht oder nur in geringem Maße absorbiert werden. Dadurch stehen sie den acetogenen Bakterien zu Verfügung, welche die Verbindungen für ihren eigenen Stoffwechsel verwerten können. Ferner können die Verbindungen im Dickdarm gespalten werden (z. B. durch Enzyme von dickdarmständigen Bakterien) und die so freigewordenen Mineralstoffe können von den acetogenen Bakterien verwertet werden. Als geeignete Verbindungen haben sich Mineralstoffverbindungen mit Phytinsäure. Tanninen, Gerbsäuren oder Oxalsäure erwiesen. Phytate können beispielsweise von bakteriellen Phytasen gespalten werden, wodurch die gebundenen Mineralstoffe (wie zum Beispiel Selen oder Wolfram) im Dickdarm verfügbar werden.

In einer weiteren Ausgestaltung der Erfindung wird vorgeschlagen, die Mineralstoffe Selen, Wolfram und/oder Molybdän und gegebenenfalls Eisen, einzeln oder in beliebiger Kombination miteinander, in einer galenischen Aufbereitung zuzuführen, die so ausgeführt ist, dass der oder die Mineralstoffe zeitlich verzögert freigesetzt werden, vorzugsweise, aber nicht ausschließlich, mit einer gleichmäßigen Wirkstofffreigabe über 48 Stunden. Dem Fachmann stehen dafür im Stand der Technik viele galenische Ausführungsmöglichkeiten zur Verfügung, von denen einige Beispiele auch in dieser Beschreibung erwähnt werden.

Dadurch, dass die durchschnittliche Verweilzeit im Dünndarm ca. 3 Stunden beträgt, werden bei einer Freigabedauer von 48 Stunden 6,25% der Mineralstoffe bereits im Dünndarm freigesetzt, und über die Dünndarmschleimhaut ins Blut des Säugetiers aufgenommen. Hierdurch kann bei Verwendung von Mineralstoffen, die bei dem Säugetier in geringen Mengen gesundheitsförderlich sind, in größeren Mengen allerdings toxisch wirken, erreicht werden, dass das behandelte Säugetier mit geringen, physiologisch wertvollen Mengen des Mineralstoffs oder der Mineralstoffe versorgt wird, die größeren Mengen jedoch im Dickdarm den acetogenen Bakterien zur Verfügung stehen. Damit zumindest ein Teil der Mineralstoffe den Dickdarm erreicht, wird vorzugsweise eine Freisetzungsdauer von mindestens 3 Stunden gewählt.

Eine weitere Ausgestaltung der Erfindung schlägt vor, den acetogenen Bakterien gezielt Stickstoff- und/oder Energielieferanten zuzuführen, um deren Wachstum zu steigern. Vorzugsweise handelt es sich um stickstoffhaltige Verbindungen oder Kohlenhydrate, die wenig oder kaum vom Darm absorbiert werden. Bevorzugt sind solche Verbindungen, die zu maximal 75 %, bevorzugt maximal 50 % und am meisten bevorzugt maximal 25 % oder weniger vom Darm absorbierbar sind. Beispiele solcher Verbindungen sind Harnstoff, Difructoseanhydrid, Rhamnose, wobei die Erfindung nicht auf diese Beispiele beschränkt sein soll.
Harnstoff wird vom menschlichen Darm nicht absorbiert und steht somit im Dickdarm zur Verfügung, wo er von den acetogenen Bakterien zum Aufbau von zur Vermehrung notwendigen Aminosäuren benutz wird.

Sind im Dickdarm des zu therapierenden Individuums bereits acetogene Bakterien mit einem ausreichenden Stoffwechselpotenzial vorhanden, kann deren Population und Stoffwechselaktivität so weit gesteigert werden, dass ein ausreichender Therapieerfolg bevorzugt ohne die Zufuhr von *in vitro* gezüchteten acetogenen Bakterien erreicht wird. Dies hat große Vorteile, da nicht autologe Stämme oft schwierig im Darm anzusiedeln sind, weil sie bestimmte Umgebungsfaktoren, auf die sie angewiesen sind, in dem zu therapierenden Individuum nicht vorfinden. Außerdem ist es nicht auszuschließen, dass nicht autologe Stämme, auch wenn sie als potenziell nicht pathogen bekannt sind, bei bestimmten Individuen pathogene Reaktionen auslösen können. Die Applizierung von autologen stämmen, die diese Nachteile nicht aufweisen, ist daher ein weiterer Aspekt der vorliegenden Erfindung. Diese Stämme können über das erfindungsgemäße Verfahren für jedes zu therapierende Individuum isoliert und dann gezüchtet werden.

In einigen Fällen kann es erforderlich sein, neben den erwähnten Mineralstoffen und/oder Vitaminen acetogene Bakterien gegebenenfalls in Kombination mit anderen Bakterienstämmen zu verabreichen. Dies ist beispielsweise dann notwendig, wenn die Stoffwechselaktivität der acetogenen Bakterien im Dickdarm nicht die erforderliche Wirksamkeit aufweist oder Stoffwechselprozesse von anderen Darmbakterien die gewünschte Acetogenese inhibieren.

Vorzugsweise werden die Mineralstoffe oder Vitamine daher mit einem hydrogenotrophen acetogenen Bakterienstamm als Kombinationspräparat oral oder rektal dem Wirt verabreicht. Hierfür geeignete Stämme sind vorzugsweise solche, die Fructose und/oder Lactose verstoffwechseln können, beziehungsweise das von dem zu therapierenden Individuum malabsorbierte Kohlenhydrat. Als geeignet haben sich insbesondere Bakterien aus der Gattung Ruminococcus, Eubacterium oder Clostridium erwiesen. Besonders bevorzugte Stämme für die vorliegende Erfindung sind *Ruminococcus hydrogenotrophicus, Ruminococcus productus* und *Clostridium coccoides, Clostridium formicoaceticum* und *Eubacterium limosum.*

Da jeder Bakterienstamm spezielle Anforderungen an seine Umgebung stellt und diese Umgebung der Dickdarm des Wirtes ist, der individuell verschiedene Umgebungsparameter aufweist, wie PH-Wert, Redoxpotenzial, bakterielle Toxine konkurrierender Bakterien, schlägt die Erfindung als vorteilhafte Ausführungsart vor, mehrere verschiedene acetogene Bakterienstämme zu applizieren, um die Wahrscheinlichkeit zu erhöhen, dass mindestens ein Stamm im Darm des Wirtes ausreichend überlebens- und vermehrungsfähig ist.

Ein besonderer Vorteil der gemeinsamen Verabreichung von acetogenen Bakterien und von Mineralstoffen, die für die Bildung der für die reduktive Acetogenese benötigten Enzyme benötigt werden, ist, dass bei zahlenmäßiger Vermehrung der acetogenen Bakterien im Darm des Wirtes genügend Mineralstoffe zur Verfügung stehen, damit die durch Teilung vermehrten Bakterien nicht jeweils nur die bei der Teilung halbierte Menge an Enzymen zum Verbleib haben, sondern durch Synthese der benötigten Enzyme die volle reduktiv acetogene Stoffwechselleistung erbringen können, und die reduktiv acetogene Stoffwechselleistung der acetogenen Bakterienpopulation im Laufe der Darmpassage deutlich ansteigt. Dadurch kann im Falle einer erforderlichen Zufuhr von acetogenen Bakterien die benötigte Menge zugeführter Bakterien signifikant reduziert und die mit der Behandlung verbundenen Kosten deutlich gesenkt werden.

Ein weiterer Vorteil der Erfindung besteht darin, dass die Verfügbarkeit von Selen, Wolfram und/oder Molybdän im Dickdarm des Säugetiers sichergestellt wird. Erst nach dem die erfindungsgemäßen Mineralstoffe in einer Form zugeführt wurden, die eine Absorption im Dünndarm weitgehend verhindert, konnte auch nach dem Absetzen der Zufuhr von frischen, in einem mineralstoffhaltigen Medium angezüchteten acetogenen Bakterien, eine ausreichende reduktiv acetogene Stoffwechseltätigkeit erhalten werden (siehe Beispiel 1, Phasen H, I und K), ohne den Wirt dem Risiko einer chronischen Schwermetallvergiftung auszusetzen.

Es wurde weiterhin festgestellt, dass beim Eintritt von größeren Mengen malabsorbierter oder maldigestierter Nährstoffe die Konzentration der von den acetogenen Bakterien gebildeten Essigsäure durch die reduktive und/oder heterotrophe Acetogenese stark ansteigen kann, was die Stoffwechselleistung der acetogenen Bakterien hemmen kann. Je nach Spezies verarbeiten die acetogenen Bakterien H2 und CO2 bis zu einer bestimmten Essigsäurekonzentration sehr effizient, während die Effizienz bei höheren Essigsäurekonzentrationen aufgrund der Produktinhibition deutlich nachlässt.

Es wurde daher nach einer Möglichkeit gesucht, die Essigsäurekonzentration zu vermindern, was erfindungsgemäß durch eine gleichzeitige Zufuhr von butyrogenen Bakterien oder durch gezielte Stimulierung der bereits im Darm vorhandenen butyrogenen Bakterien erreicht werden konnte. Diese Bakterien verstoffwechseln einen beträchtlichen Anteil der anfallenden Essigsäure zu Buttersäure, wodurch sie die Essigsäurekonzentration senken und dadurch sowohl das Wachstum als auch die Stoffwechselleistung der acetogenen Bakterien deutlich erhöhen.

Es wird daher in einer weitergehenden Ausgestaltung der Erfindung vorgeschlagen, dass die der Erfindung zugrundeliegende Zusammensetzung zusätzlich butyrogene Bakterien, (z. B. Fusobacterium, Faecalibacterium, Eubacterium, mit oder ohne Mineralstoffen bzw. Vitaminen) enthält. Besonders vorteilhaft sind Stämme, die vorwiegend Essigsäure zu Buttersäure verstoffwechseln. Ein besonders vorteilhaftes Beispiel ist *Faecalibacterium prausnitzii.*

Für die Fälle, in denen auf eine Verabreichung von Mineralstoffen und Vitaminen sowie Energie- und Stickstoffquellen für eine Förderung der acetogenen Bakterien verzichtet werden kann, wird eine Zusammensetzung vorgeschlagen, die butyrogene Bakterien enthält.

In einer weiteren Ausgestaltung der vorliegenden Erfindung enthält die Zusammensetzung Mineralstoffe, Vitamine und/oder Stickstoffdonatoren und gegebenenfalls durch die butyrogenen Bakterien verwertbare zusätzliche Energie- und Kohlenstoffquellen (z. B. Kohlenhydrate) in dieser Kombination ist es möglich, die butyrogenen Bakterien in ihrem Wachstum und ihrer Stoffwechselaktivität zu fördern und durch den damit verbundenen Umsatz von Essigsäure eine optimale Aktivität der Population acetogener Bakterien im Dickdarm zu erreichen. Vorzugsweise sollten die Stickstoffverbindungen und Kohlenhydrate nicht oder nur wenig vom Darm absorbiert werden, was entweder durch die für den Wirtsorganismus vorwiegend nichtdigestierbare Natur dieser Zusatzstoffe erreicht werden kann oder durch die Verwendung einer entsprechenden galenischen Aufbereitung.

Die Begünstigung einer Ansiedelung bzw. Erhaltung und Regenerierung acetogener Baktenen, beziehungsweise deren Stoffwechselaktivierung im Dickdarm hat sich, wie anfangs erwähnt, insbesondere zur Behandlung und Therapie von abdominalen Beschwerden als wirksam herausgestellt.

Die positiven Auswirkungen der erfindungsgemäßen Zusammensetzung beschränken sich jedoch nicht nur auf die Behandlung von abdominalen Beschwerden, sondern sind auch bei einer prophylaktischen Anwendung erkennbar. Es können nicht menschliche Säugetiere wie Hund, Katze, Schweine, Rind, Schaf, Ziege oder andere Nutztiere und der Mensch mit der erfindungsgemäßen Zusammensetzung behandelt werden.

Die Verabreichung der Zusammensetzung erfolgt vorzugsweise oral über die zuvor erwähnten magen- und dünndarmresistenten Kapseln. Alternativ kann die Zusammensetzung auch als Zäpfchen rektal oder eine Hydro-Colon-Therapie verabreicht werden.

Es hat sich gezeigt, dass bei hohen Mineralstoffspiegeln im Darminhalt die Mineralstoffe teilweise auch von der Dickdarmschleimhaut aufgenommen werden, was die Toxizitätsgefahr erhöht und die Verfügbarkeit für die Darmbakterien vermindert. Deshalb schlägt die Erfindung in einer bevorzugten Ausführungsform vor, dass die Freisetzung der Mineralstoffe verzögert wird, z.B. als Bestandteil des Matrixkerns einer dünndarmresistent beschichteten Tablette, einer Kapsel mit Diffusionsmembran, einer Kapsel mit definierter Mikroöffnung und osmosegesteuerter Freisetzung, oder andere geeignete galenische Aufbereitungen, und dafür sorgt, dass die Mineralstoffspiegel am Dickdarmanfang nicht zu hoch sind, und sich im Laufe der Passage des Darminhalts durch den Dickdarm nicht zu sehr absenken, und somit immer ein möglichst optimaler Mineralstoffspiegel vorhanden ist. Somit ergibt sich am Anfang des Dickdarms eine geringere unerwünschte Absorption und am Ende der Darmpassage ein ausreichend hoher Mineralstoffspiegel um eine effektive reduktiv acetogene Stoffwechselleistung der acetogenen Bakterien sicherzustellen.

Bevorzugt werden abdominale Beschwerden behandelt bzw. vorgebeugt, die durch folgende gastrointestinale Störungen verursacht werden, bzw. würden: Fructosemalabsorption, Fructoseintoleranz, Lactoseintoleranz, Irritiertes Bowel Syndrom (Reizdarmsyndrom), Disaccharasemangel, Trehalasemangel, Kurzdarmsyndrom, Morbus Crohn, Dreimonatskoliken, exokrine Pankreasinsuffizienz, Gallensäuremangel, Magensäuremangel, antibiotikainduzierte Dysbiose der Darmflora, Zöliakie, Sprue, Gluten-Unverträglichkeit, Histaminintoleranz.

Auch während oder nach einer Antibiotikatherapie, Hydro-Colon-Therapie und Darmlavagen oder gastrointestinalen Infektionen, zum Beispiel mit Enteroviren oder pathogenen Enterobakterien, kann eine Verabreichung der beschriebenen erfindungsgemäßen Zusammensetzungen empfohlen sein. Insbesondere bei einer Antibiotikatherapie wird durch den Wiederaufbau der durch das/die Antibiotikum(ka) gestörten Darmflora sichergestellt, dass sich eine ausreichende Anzahl von acetogenen und/oder butyrogenen Bakterien ansiedelt, um Abdominalbeschwerden nach dem Verzehr von unverdaulichen Nahrungsbestandteilen (z.B. Ballaststoffen) oder bei Malabsorptionen und Maldigestionen zu verhindern oder zumindest zu lindern. Es ist daher vorgesehen, die dieser Erfindung zugrundeliegenden Zusammensetzungen auch während und/oder nach einer Antibiotikatherapie zur Unterstützung des Darmflorawiederaufbaus zu verwenden.

Eine Kombination mit üblichen Milchsäurebakterien wie Laktobazillen, Bifidobakterien, Enterokokken und nicht pathogenen Enterobakterien wie ausgesuchte Stämme von *Escherichia coli* ist ebenfalls möglich und im Einzelfall je nach Zustand der Darmflora und den auftretenden Symptomen anzuwenden.

Bei Verabreichung an weibliche Betroffene enthält die erfindungsgemäße Zusammensetzung bevorzugt Selen und/oder Wolfram. Wolfram hat sich als besonders vorteilhaft zur Behandlung und Prophylaxe abdominaler Beschwerden und Störungen beim Menschen herausgestellt. Selen wird natürlicherweise schon im oberen Dünndarm beim Menschen absorbiert und kann daher (ohne externe Versorgung) nicht den acetogenen Bakterien im Dickdarm zur Vermeidung oder Vorbeugung der Gasbildung zur Verfügung stehen. Ferner wurde festgestellt, dass Wolfram von weiblichen Betroffenen im starken Maße absorbiert wird, was ein Grund ist, warum weibliche Betroffene sehr oft über eine stärkere Gasbildung klagen, als männliche Betroffene.

Die Erfindung betrifft ferner die Verwendung eines butyrogenen Bakterienstamms zur Behandlung und/oder Vorbeugung abdominaler Beschwerden in einem Säugetier, die von einer verminderten reduktiv acetogenen Stoffwechselleistung begleitet sind. Für diese Zusammensetzungen gelten die für die acetogenen Bakterien vorstehend beschriebenen Varianten und Ausführungsformen entsprechend.

Für eine Ansiedelung von zugeführten butyrogenen Bakterien im menschlichen Darm spielt die Generationszeit bei der Verstoffwechselung von Essigsäure und Kohlenhydraten eine große Rolle. Für den therapeutischen Erfolg ist die Stoffwechseikapazität unter den jeweils herrschenden Bedingungen zu beachten, was durch die Essigsäureabbaukapazität biochemisch über bekannte Verfahren gemessen werden kann. Beispielhafte Gattungen von butyrogenen Stämmen sind: Fusobacterium, Faecalibacterium, Eubacterium, Clostridium.

Um solche vorteilhaften butyrogenen Bakterienspezies oder Bakterienstämme zu finden wird deshalb in einer weiteren Ausführungsform der Erfindung vorgeschlagen, diese aus menschlichen Fäkalien zu isolieren, da dort autologe butyrogene Bakterien vorhanden sind, welche die erforderliche Überiebensfähigkeit im menschlichen Darm aufweisen, Im Allgemeinen werden in der Mikroflora des Darmes nur solche butyrogene Bakterien überleben, welche über die entsprechenden Stoffwechselaktivitäten verfügen und sich gegenüber anderen Bakterien durchgesetzt haben. Auf diese Weise kommt eine natürliche Selektion von Bakterienstämmen mit den erwünschen Stoffwechselkapazitäten zustande.

In einer bevorzugten Ausführungsform wird bei dem erfindungsgemäßen Verfahren eine Verdünnungsreihe mit den isolierten butyrogenen Bakterien in einem geeigneten Lösungsmittel angelegt und auf ein anaerobes Wachstumsrnedium, das Essigsäure als einzige Energiequelle sowie einen Essigsäureindikator besitzt, aufgebracht. Vorzugsweise erfolgt die Aufzucht dieser isolierten butyrogenen Bakterien unter anaeroben Bedingungen, gegebenenfalls unter einer Stickstoffatmosphäre.

Die Stuhlproben sollten vorzugsweise von Menschen stammen, die eine dem Beschwerdabild zugrundeliegende Ursache aufweisen, jedoch total oder zumindest weitgehend symptomfrei sind. Nach Born et al. weisen nur ca. 40% der Fructosemalabsorber gastrointestinale Symptome auf. Der Erfinder vermutet, dass die symptomfreien Malabsorber über ausreichend effizient arbeitende acetogene und/oder butyrogene Bakterien verfügen, so dass solche symptomfreien Malabsorber bevorzugt als Bakterienlieferanten in Frage kommen. Dies erhöht die Wahrscheinlichkeit, solche butyrogenen Bakterien zu finden, die an die Verwertung von Essigsäure, die bei diesen Menschen durch die acetogenen Bakterien produziert wird, adaptiert sind und diese effektiver zu Buttersäure abbauen können.

Die sich durch eine kurze Generationszeit (als große Zellkolonien sichtbar) und einen starken Essigsäureverbrauch (Indikatoransprechen) auszeichnenden Kulturen werden bevorzugt nach ihrer Generationszeit bei Verstoffwechselung von Essigsäure isoliert und charakterisiert. Analog zu der isolierung bevorzugter butyrogener Bakterienstämme wird vorgeschlagen mit entsprechenden Verfahren bevorzugte acetogene Bakterienstämme zu isolieren. Im Gegensatz zur Isolierung butyrogener Stämme wird dem Medium als einzige Energie- und Kohlenstoffquelle H2 und CO2 in der Gasphase bereitgestellt. Als Indikator dient ein Säureindikator, der anzeigt, wie effektiv die jeweiligen Bakterien die angebotenen Gase zu Essigsäure verstoffwechseln können. Als Indikatoren können unter Anderem Thymolblau, Methytorange, Mettyylrot oder Phenolphtalein dienen.

Bei einem Teil der Menschen, die an gastrointestinalen Störungen leiden, äußern sich die Beschwerden nicht oder nur zum Teil in durch Gasbildung verursachten Blähungen, sondern auch oder hauptsächlich durch Durchfälle oder Darmkrämpfe, die zum Beispiel durch die osmotische Wirkung von unverdauten Nahrungsbestandteilen verursacht werden. Dadurch dass die acetogenen Bakterien auch heterotrophen Stoffwechsel betreiben, können sie auch in diesen Fällen die Beschwerden lindem. Durch die höheren Flüssigkeitsmengen, die durch die: Osmosewirkung in den Darm gelangen, wird die Darmpassagezeit jedoch oft so stark verringert, dass die heterotrophe Stoffwechsefleistung der acetogenen Bakterien nicht ausreicht um die osmotisch wirkenden unverdauten Nahrungsbestandteile schnell genug zu verstoffwechseln.

Es ist bekannt, dass Enterobakterien, vornehmlich Escherichia Coli, Kohlenhydrate viel schneller verstoffwechseln können als die meisten anderen in der Darmflora vorkommenden Bakterien, und so osmotische Durchfälle abmildern oder gar verhindern können. Allerdings verstoffwechseln diese Bakterien die von ihnen als Nahrungssubstrat verwendeten Kohlenhydrate vorwiegend zu Gasen (H2 und CO2), was dann wiederum zu Beschwerden durch Blähungen führen kann.

Die Erfindung schlägt weiterhin eine bevorzugte Ausführung vor, in der zusätzlich Enterobakterien, vorzugsweise der Gattung Escherichia Coli, verabreicht werden, um die unverdauten Nahrungsbestandteile durch die Enterobakterien zu Gasen verstoffwechseln zu lassen, damit diese Gase dann von den acetogenen Bakterien zu Essigsäure verstoffwechselt werden können, um entweder von der Darmschleimhaut absorbiert zu werden, oder den butyrogenen Bakterien als Substrat zur Verfügung zu stehen. Diese Ausführung macht sich die gasabbauende Wirkung einer der vorstehend beschriebenen erfindungsgemäßen Zusammensetzungen zunutze um die bei einer konventionellen Durchfallbehandlung durch Enterobakterien entstehende Gasbildung, und somit die Nebenwirkungen Blähungen und Meteorismus zu behandeln oder ihnen vorzubeugen.

Vorzugsweise erfolgt die Verabreichung in einer kombinierten galenischen Aufbereitung.

Die nachfolgenden Beispiele veranschaulichen die Erfindung im Detail, welche jedoch nicht auf diese beschränkt sein soll.

### Wege zur Ausführung der Erfindung und gewerbliche Verwertbarkeit

### Beispiele

### Beispiel 1:

Versuchsperson: Westeuropäer, männlich, 35 Jahre alt, normalgewichtig, Fructosemalabsorption mit Blähungen als dominantes Symptom.

### Vorbereitung:

Herstellung der Bakterienpellets

### Kultivierungsmedium:

Für die Vorkultur wurde ein modifiziertes Kochfleischmedium (RACh nach Nollet et al.) verwendet. Zusätzlich wurden 0,1 mmol Natriumselenit und 0,1 mmol Natriumwolframat zugesetzt. Außerdem wurde 1% Fructose zugefügt.

Das vorreduzierte Medium wurde in 15ml Hungate-Röhrchen gefüllt, mit 80% H2 und 20% CO2 als Gasphase bedeckt und die gefriergetrockneten Pellets vom Stamm *Ruminococcus hydrogenotrophicus* DSM 10507 (Deutsche Sammlung von Mikroorganismen und Zellkulturen GmbH). Die Vorkulturen wurden 48 Stunden bei 37°C bebrütet.

Für die zu zentrifugierenden Hungate-Röhrchen wurden keine Fleischpartikel sondern nur die Fleischbrühe zugesetzt.

Das Medium wurde unter anaerober Atmosphäre (N2) in 10ml Hungate-Röhrchen gefüllt, mit 80% H2, 20% CO2 geflutet und mit 0.5ml der Vorkultur inkubiert. Die Bebrütung erfolgte bei 37°C für 36 Stunden. Danach wurden die Bakterien durch Zentrifugieren abgetrennt und gefriergetrocknet. Anschließend erfolgte, ebenfalls unter anaerober Atmosphäre, die Beschichtung mit Eudragit FS 30 D.

### Phase A:

### Basisdiät:

Streng Fructosearm (< 1 g/Tag)
Verwendete Lebensmittel: Reis, Reiswaffeln, Reisflocken, Putenfleisch, Eier, Milch, Joghurt, zuckerfreies Weizen- Weißmehlbrot, Lachs geräuchert, Yamswurzel, Kartoffeln gewässert, Rapsöl, Salz, Pfeffer.
Nahrungsergänzungsmittel, Multivitaminpräparat (Multiblonata Tropfen), Folsäure (Ratiopharm), Zink (Zinkorot). Mit Hilfe von zweimal täglich 7 g Flohsamenschalen und 5 g Polyethyleng4,kal 4000 wurde die Stuhlkonsistenz so eingestellt, dass eine Defäkation über ein Charrière 40 Darmrohr möglich war. Die Basisdiät wurde für 4 Wochen eingehalten. Während dieser Zelt war die Testperson beschwerdefrei.

### Phase B:

### Fructosebelastungsdiät:

Zu oben beschriebener Basisdiät wurden 10g Fructose pro Tag addiert, jeweils 2 g bei jeder der 5 Mahlzeiten täglich als Pulver direkt in die Speisen gerührt. Während Phase B klagte die Versuchsperson über heftige Beschwerden (Blähungen und Bauchkrämpfe). Die Fructosebelastungsdiät wurde bis zum Ende des Experiments durchgeführt. Phase B dauerte 10 Tage.

### Phase C:

### Applikation von acetogenen Bakterien:

Verwendet wurde der Stamm *Ruminococcus hydrogenotrophicus* DSM 10507 (Deutsche Sammlung von Mikroorganismen und Zellkulturen GmbH). Die mit Eudragit FS 30 D beschichteten Pellets wurden im Laufe der Hauptmahlzeiten (dreimal täglich ein beschichtetes Pellet) unzerkaut geschluckt. Ab dem zweiten Tag von Phase C bemerkte die Versuchsperson eine deutliche Verringerung der Beschwerden, die bis zum Ende der Phase C anhielt. Phase C wurde für 10 Tage durchgeführt.

### Phase D:

### Absetzen der acetogenen Bakterien.

Es wurden nun keine acetogenen Bakterien mehr zugeführt, sondern nur noch die Fructosebelastungsdiät fortgeführt. Ab dem dritten Tage der Phase D klagte die Versuchsperson erneut über intensivere Beschwerden, am siebten Tag wurde deshalb beschlossen direkt zu Phase E zu wechseln (geplant waren ebenfalls 10 Tage für Phase D).

### Phase E:

### Applikation von acetogenen Bakterien und Mineralstoffen:

Die acetogenen Bakterien wurden analog zu Phase C zugeführt. Zusätzlich wurden pro Hauptmahlzeit 10 µg Selen in Form von Natriumselenit und 20 µg Wolfram in Form von Natriumwolframat als Pulver direkt in die Mahlzeit eingerührt. Ab dem zweiten Tag von Phase E bemerkte die Versuchsperson wieder die deutliche Verringerung der Beschwerden, die auch wieder zum Ende dieser Phase anhielt. Phase E wurde für 10 Tage durchgeführt.

### Phase F:

Absetzen der acetogenen Bakterien unter Fortführung der Mineralstoffapplikation. Es wurden pro Hauptmahlzeit 10 µg Selen in Form von Natriumselenit und 20 µg Wolfram in Form von Natriumwolframat als Pulver direkt in die Mahlzeit eingerührt. Ab dem dritten Tage der Phase F klage die Versuchsperson erneut über intensivere Beschwerden. Wiederum am siebten Tag wurde deshalb beschlossen, erneute acetogene Bakterien zuzuführen (geplant waren ebenfalls 10 Tage für Phase F).

### Phase G:

Erneute Applikation von acetogenen Bakterien:
Phase G wurde analog zu Phase C durchgeführt. Der Beschwerdeverlauf war entsprechend.
Phase G wurde für 14 Tage durchgeführt bis Phase H entwickelt war.

### Phase H:

Applikation von acetogenen Bakterien und Mineralstoffen in dünndarmresistentem Überzug: Die acetogenen Bakterien wurden analog zu Phase C zugeführt. Zusätzlich wurden pro Hauptmahlzeit 10 µg Selen in Form von Natriumselenit und 20 µg Wolfram in Form von Natriumwolframat zugeführt. Die Mineralsalze wurden zusammen mit Methylcellulose zu Tabletten gepresst und ebenfalls mit Eudragit FS 30 D beschichtet, allerdings mit höherer Schichtdicke, um nicht nur Magensaftresistenz zu erreichen, sondern durch die längere Auflösungszeit der dickeren Beschichtung die Mineralsalze erst im Dickdarm freizusetzen. Ab dem zweiten Tag von Phase H bemerkte die Versuchsperson, dass die Beschwerden, die durch die Applikation der acetogenen Bakterien schon deutlich verringert waren, gänzlich verschwanden. Phase H wurde für 10 Tage durchgeführt.

### Phase I:

Absetzen der acetogenen Bakterien unter Fortführung der Mineralstoffapplikation mit dünndarmresistentem Überzug. Es wurden analog zu Phase H pro Hauptmahlzeit 10 µg Selen in Form von Natriumselenit und 20 µg Wolfram in Form von Natriumwolframat mit dünndarmresistenter Beschichtung aus Eudragit FS 30 D zugeführt. Es wurde kein erneutes Auftreten von Beschwerden beobachtet. Phase I wurde für 10 Tage durchgeführt.

### Phase J:

### Absetzen der Mineralstoffe

Analog zu Phase B wurde nur die Fructosebelastungsdiät zugeführt. Ab dem vierten Tag traten erneut Beschwerden auf, ab dem achten Tag mit voller Intensität wie bei Phase B und F.

### Phase K:

### Mineralstoffapplikation mit dünndarmresistentem Überzug.

Analog zu Phase I wurden pro Hauptmahlzeit 10 µg Selen in Form von Natriumselenit und 20 µg Wolfram in Form von Natriumwolframat mit dünndarmresistenter Beschichtung aus Eudragit FS 30 D zugeführt. Ab dem zweiten Tag ließen die Beschwerden nach und ab dem fünften Tag war die Versuchsperson praktisch beschwerdefrei. Phase K wurde für 10 Tage durchgeführt.

### Phase L:

### Zufuhr butyrogener Bakterien

Da die Gasmenge bei Phase I immer noch signifikant höher war als bei Phase A wurde nach einem Weg gesucht, die Arbeitsleistung der acetogenen Bakterien nochmals zu erhöhen. Da interspezieller Nährstofftransfer eine Stoffwechselstimulierung bewirken kann, wurden analog zu den acetogenen Bakterien zusätzlich zu den dünndarmresistent überzogenen Mineralstoffen noch butyrogene Bakterien verabreicht und zwar *Faecalibacterium prausnitzii.*

Eine subjektive Verbesserung der Symptome wurde allerdings nicht festgestellt, wobei die Darmgasmenge weiter gesenkt werden konnte, was darauf schließen lässt, dass die Applikation butyrogener Bakterien die tolerierbare Menge der malabsorbierten Kohlenhydrate erhöhen kann.

### Phase M:

### Zufuhr unverkapselter Mineralstoffe in größerer Menge

Um zu untersuchen, ob die Applizierung von unverkapselten Mineralstoffen in höherer Dosierung (um die Absorption im Dünndarm auszugleichen) wirksam sind, wurden nochmals Mineralstoffe als unverkapseltes Pulver verabreicht, allerdings die Mineralstoffmenge pro Mahlzeit gegenüber der nicht erfolgreichen Phasen E+F verzehnfacht, also pro Mahlzeit 100 µg Selen in Form von Natriumselenit und 200 µg Wolfram in Form von Natriumwolframat.
Es zeigte sich eine bessere Wirksamkeit als ohne Mineralstoffzufuhr, jedoch weniger gut als mit dünndarmresistenter Verkapselung, trotz der größeren Menge.
Für kurzfristige Applikationen, bei denen keine größere Gefahr besteht, dass sich die zu hohen Mineralstoffmengen im Körper anreichern, scheint auch eine solche, höherdosierte, unverkapselte Anwendung sinnvoll.

### Phase N:

### Ersatz von Wolfram durch Molybdän, Mangan und Vanadium

Um die Effektivität von Molybdän, Mangan und Vanadium zu untersuchen wurde zuerst Phase D wiederholt, und sodann eine Verabreichung wie in Phase F durchgeführt, wobei die 20µg Wolfram durch 20µg Molybdän 20µg Vanadium und 20µg Mangan ersetzt wurden.
Es zeigte sich eine leicht bessere Wirksamkeit als ohne Mineralstoffzufuhr, jedoch weit weniger gut als mit Wolfram.

### Phase O:

### Untersuchung der Wirksamkeit von unverdaulichen Kohlenhydraten zur Populationserhaltung der acetogenen Bakterien

Zu Anfang von Phase O wurden analog Phase H für 10 Tage acetogene Bakterien und Selen plus Wolfram appliziert. Danach wurde für 20 Tage auf die fast fructose- und ballaststofffreie Basisdiät umgestellt, keine acetogenen Bakterien mehr appliziert, die Gabe von Selen und Wolfram jedoch weitergeführt. Dann wurde wieder auf die Fructosebetastungsdiät umgestellt, unter Fortführung der Mineralstoffapplikation. Es wurde eine deutlich höhere Gasmenge als z.B. bei Phasen I und K.
Danach wurde noch einmal Phase H für 10 Tage wiederholt, und wiederum für 20 Tage auf die Basisdiät umgestellt. Allerdings wurde zusätzlich zu Selen und Wolfram über den Tag verteilt 5g Difructoseanhydrid (DFA-III) verabreicht. Bei der anschließenden Umstellung auf die Fructosebeiastungsdiät konnte wieder eine ähnlich geringe Gasmenge wie bei Phase I erreicht werden. Das DFA-III konnte während der Fructose- und Baltaststoffkarenz die Population der acetogenen Bakterien weitgehend erhalten. Dies ist eine Möglichkeit auch bei Ungleichmäßigkeiten in der Diät auf eine dauerhafte Zufuhr von acetogenen Bakterien nicht immer angewiesen zu sein.

Am letzten Tag jeder Phase wurde die Darmgasmenge gemessen. Dazu wurde ein verschließbares Doppelballondarmrohr verwendet, das jeweils für 24 Stunden getragen wurde und an das während der ganzen Nacht und tagsüber bei Bedarf ein Auffangbeutel für Darmgase und Stuhl angeschlossen wurde.

Bei den Messungen wurden folgende Gasmengen gemessen:

| Phase | Beschreibung | Gasmenge in Liter |
|---|---|---|
| A | Basisdiät | 0,25 |
| B | Fructosebeiastungsdiät | 3,45 |
| C | Applikation von acetogenen Bakterien | 0,77 |
| D | Absetzen der acetogenen Bakterien | 3,10 |
| E | Applikation von acetogenen Bakterien und Mineralstoffen | 0,51 |
| F | Absetzen der acetogenen Bakterien, Fortführung der Mineralstoffapplikation | 2,93 |
| G | Erneute Applikation von acetogenen Bakterien | 0,70 |
| H | Zusätzlich Applikation Mineralstoffen in dünndarmresistentem Überzug | 0,35 |
| I | Absetzen der acetogenen Bakterien, Fortführung der Mineralstoffapplikation | 0,42 |
| J | Absetzen der Mineralstoffe | 3,20 |
| K | Erneute Applikation von Mineralstoffapplikation mit dünndarmresistentem Überzug | 0,45 |
| L | Zusätzlich Applikation butyrogener Bakterien | 0,30 |
| M | unverkapselter Mineralstoffe in höherer Dosierung | 1,45 |
| N | Ersatz von Wolfram durch Molybdän, Mangan und Vanadium | 2,20 |
| O 1 | Nach Fructosepause | 0,78 |
| O 2 | Nach Fructosepause mit Kohlenhydraten zur Populationserhaltung | 0,48 |

Die gemessenen Gasmengen korrelierten deutlich mit den aufgetretenen Symptomen. Durch die Zugabe von acetogenen Bakterien (Phasen C, E, G, H) konnte eine deutliche Reduktion der Gasbildung erzielt werden, die allerdings nach Absetzen der Zufuhr nicht anhielt (Phasen D und F). Durch Zugabe von für die reduktive Acetogenese notwendigen Mineralstoffen in einer dünndarmresistenten Aufbereitung konnte die Gasbildung weiter reduziert werden (Phase H) und auch nach der Beendigung der Zufuhr von acetogenen Bakterien aufrecht erhalten werden (Phasen I und K). Eine Zugabe von butyrogenen Bakterien konnte die Gasbildung nochmals reduzieren (Phase L). Eine Reduktion der Gasbildung ist demnach feststellbar:
bei Verabreichung der Mineralstoffe und/oder Vitamine in dünndarmresistenter Aufbereitung
bei Verabreichung acetogener Bakterien
bei Verabreichung butyrogener Bakterien
bei einer kombinierten Verabreichung von Mineralstoffen in dünndarmresistenter Aufbereitung und/oder Vitaminen und acetogenen oder butyrogenen Bakterien.
bei Verabreichung der Mineralstoffe und/oder Vitamine in dünndarmresistenter Aufbereitung und butyrogenen Bakterien.
bei Verabreichung der Mineralstoffe in hohen Dosen, wobei die Intoxikationsrisiken beachtet werden müssen.

### Beispiel 2:

Folgende Stoffe werden in Pulverform gemischt:
40 µg Natriumwolframat, 30 µg Natriumselenit, 50 µg Mangansulfat, 50 µg Natriummolybdal, 5 mg Eisensulfat, 50 µg Nickelsulfat, 50 mg Hefeextrakt und 150 mg Hydroxymethylcellullose.

Danach wird das Gemisch in Tablettenform gepresst und mit Eudragit L 100 und Eudragit S 100 im Verhältnis 80 : 20 beschichtet. Die Dicke der Beschichtung wird so eingestellt, dass die Inhaltsstoffe 3 Stunden nach dem Erreichen eines pH-Wertes von > 6,5 freigesetzt werden. Alternativ kann die Beschichtung mit Eudragit FS 30 D erfolgen.

Bei einer Einnahme nach mindestens 3 Stunden Nüchternheit (4 Stunden nach fett- und eiweißreicher Nahrung) und mindestens 30 Minuten vor der nächsten Nahrungsaufnahme (üblicherweise morgens direkt nach dem Erwachen) wird sichergestellt, dass die Wirkstoffe erst nach dem Transit durch den Dünndarm freigesetzt werden.

Man beobachtete eine deutliche Abnahme der Gasbildung im Dickdarm. Zur Gasmessung wurde wie in Beispiel 1 ein verschließbares Doppelballondarmrohr verwendet, das jeweils für 24 Stunden getragen wurde, und an das während der ganzen Nacht und tagsüber bei Bedarf ein Auffangbeutel für Darmgase und Stuhl angeschlossen wurde.

Des Weiteren wurden Stuhlproben des behandelten Patienten vor und nach Verabreichung der Tabletten entnommen. Dabei konnte eine Zunahme der Menge an acetogenen Bakterien nach dem Beginn der Therapie festgestellt werden und eine deutlich verringerte Gasbildung bei Inkubierung der Stuhlprobe unter Zugabe von Fructose (1g pro 100g Stuhlmenge).
Weiterhin wurde festgestellt, dass bei einer Inkubierung des Stuhls ohne Nährstoffizugabe, unter Begasung mit 80% H2 und 20% CO2, der Gasdruck nach der Therapie sank, wogegen er bei vor der Therapie entnommenen Stuhlproben sogar angestiegen war.

### Beispiel 3

Stunlproben von 20 Personen, welche nach eigenen Angaben keine Blähungsprobleme mit üblicherweise blähenden Lebensmitteln (Zwiebeln, Kohl, Hülsenfrüchte) hatten, wurden homogenisiert und auf jeweils 6 Inkubationsbehälter aufgeteilt. Die Inkubationsbehälter wurden allesamt mit 1g/100g Stuhlmenge an Fructose versetzt, und Zusätzlich mit 1. 20µg Selen; 2. 40µg Wolfram; 3. 20µg Selen plus 40µg Wolfram: 4. 20µg Selen plus 40µg Vanadium; 5. 20µg Selen plus 40µg Molybdän; 6. 20µg Selen plus 40µg Wolfram plus 1 Tropfen Multivitaminlösung "Multibionta"; 7. 20µg Selen plus 40µg Wolfram plus 1 Tropfen Multivitaminlösung "Multibionlaplus 1 mg Eisen plus 20µg Nickel. Ein Kontrollbehälter wurde nur mit Fructose versetzt.

Die Inkubationsbehälter wurden mit einer Gasmengenmesseinheit versehen gasdicht verschlossen und bei 37° inkubiert.

Die folgenden Gasmengen wurden gemessen:

| | |
|---|---|
| Kontrolle | 150ml |
| Se | 110ml |
| W | 100ml |
| Se+W | 20ml |
| Se+V | 100ml |
| Se+Mo | 85ml |
| Se+W+Vitamin | 15ml |
| Se+W+Vitamin+Fe+Ni | 11ml |

Mit Selen und Wolfram war eine gute Reduktion der Gasmenge festzustellen, die durch Zusatz von Vitaminen und Eisen plus Nickel noch verbessert werden konnte. Auch alleine zeigten Selen oder Wolfram signifikante Gasreduktionen.
Die Reduktion durch Vanadium und Molybdän fiel im Schnitt deutlich geringer aus, wobei der Haupteinfluss diese Mineralstoffe durch eine gute Reduktion bei zwei der 20 Stuhlproben zustande kam, und sie bei den anderen Stuhlproben fast wirkungslos waren. Es liegt nahe, dass die acetogenen Bakterienstämme in diesen Stuhlproben in ihren Enzymen diese Metalle statt dem Wolfram verwenden. Dies lässt sich auch daraus schließen, dass Wolfram bei diesen Proben eine nicht ganz so überlegene Wirkung zeigte. Um die Erfolgsquote zu erhöhen erscheint es vorteilhaft, zu dem Wolfram noch Vanadium und/oder Molybdän dazuzugeben. Außerdem erscheint es vorteilhaft, noch Vitamine, Nickel und Eisen hinzuzufügen, da diese die Gasbildung weiter reduzieren konnten.

### Beispiel 4 kontinuierliche Freisetzung:

Folgende Stoffe werden in Pulverform gemischt:
90 mg Natriumwolframat, 50 mg Natriumselenit, 50 mg Mangansulfat, 50 mg Natriummolybdat. 5 g Eisensulfat, 50 mg Nickelsulfat, 50 g Hefeextrakt, 50 g Harnstoff.

90% dieses Gemisches werden wiederum vermischt mit 150 g dibasischem Kafziumphosphat (Encompress (R)), 20 g Polyvinylpyrrolidon, 120 g Hydroxypropylcellulose, und durch ein mesh 40 Sieb passiert, und mit 90 g Hydroxypropylmethylcellulose-phtalat und 10g Beta-cyclodextrin welche zusammen in einer ausreichenden Menge Aceton aufgelöst wurden, granuliert.
Die feuchte Masse wird getrocknet und durch ein mesh 30 Sieb passiert um das Granulat zu erhalten.

Analog zu den schon beschriebenen Vorbereitungen zu Beispiel 1 werden acetogene Bakterien angezüchtet. Es werden die Stämme *Ruminococcus hydrogenotrophicus* DSM 10507 und *Ruminococcus productus* DSM 2950 und *Ruminococcus productus* DSM 3507 und Clostridium Coccoides DSM 935 (Deutsche Sammlung von Mikroorganismen und Zellkulturen GmbH).

Die gefriergetrockneten lyophilisierten Bakterien aller 3 Stämme (jeweils 10*10¹² koloniebildende Einheiten) werden unter anaeroben Bedingungen mit den restlichen 10% des Pulvergemisches vermischt und zusammen mit dem Granulat in 1000 HPMC-Kapseln abgefüllt, die Kapseln verschlossen und mittels Eudragit FS 30 D in einer Schichtdicke von 8mg/cm² Dünndarmresistent beschichtet.
Die Verwendung erfolgt analog Beispiel 1, 2, 5 bis 7 oder 9 bis 12.

### Beispiel 5

Folgende Stoffe werden in Pulverform gemischt:40 µg Natriumwolframat, 30 µg Natriumselenit und 200 mg hochdisperses Siliziumdioxid. Das Gemisch wird in Hydroxypropylmethylcellulose-Kapseln abgefühlt, und die Kapseln mit 8mg/cm² Eudragit FS-30 D beschichtet, so dass sie sich beim Erreichen des Dickdarms auflösen.
Eine Frau, 28 Jahre, normalgewichtig, laktoseintolerant mit einer maximalen beschwerdsfreien Laktaseverträglichkeit von 1,5g Laktose pro Tag (entsprechend 60g Hüttenkäse) wird behandelt mit den beschichteten Kapseln, in einer Dosis von jeweils einer Kapsel zu jeder der drei Hauptmahlzeiten pro Tag. Ab dem dritten Tag der Behandlung wird zusätzlich zu den mit der üblichen Diät aufgenommenen 1,5g Laktose pro Tag jede Hauptmahlzeit mit Laktose angereichert, beginnend mit 0,5g und mit einer Steigerung von 0,5g pro Tag und Hauptmahlzeit. Nachdem auch der vierte Tag (der 6. Tag seit Beginn der Behandlung) beschwerdefrei ist, wird die erreichte Tagesmange an Laktose (1,5g plus 3*2g) beibehalten und die Behandlung mit Leerkapseln (als Placebopräparat) fortgeführt. Ab dem zweiten Tag der Placebogabe treten abdominale Beschwerden in Form von Blähung auf, wie sie die Testperson aus der Zeit ohne Behandlung bei zuviel Laktoseverzehr kennt. Am Abend des dritten Tags der Umstellung auf Placebo bittet die Patientin den Test abbrechen zu dürfen

### Beispiel 6

Ein Mann, 40 Jahre, mit exokriner Pankreasinsuffizienz, beschwerdefrei ab einer Dosierung von 40000 Einheiten Pankreatin pro Hauptmahlzeit (1 Kapseln Kapsel des Pankreatinpräparates Kreon 40000 der Firma Solvay) wird behandelt mit den beschichteten Kapseln analog Beispiel 5 in einer Dosis von jeweils einer Kapsel zu jeder der drei Hauptmahlzeiten pro Tag. Ab dem dritten Tag wird die Pankreatindosis reduziert (zwei Kapseln Kreon 10000 pro Hauptmahlzeit), der Stuhl wird etwas weicher, der Patient bleibt beschwerdefrei. Ab dem fünften Tag wird die Pankreatindosis nochmals reduziert (eine Kapsel Kreon 10000 pro Hauptmahlzeit). Der Stuhl wird noch weicher und leicht fettig glänzend, jedoch nicht als unangenehm empfunden. Blähungsbeschwerden, die den Patienten ohne oder mit solch geringer Pankreatinversorgung üblicherweise stark stören, bleiben weiterhin aus. Die kostenintensive Enzymsubstitution kann deutlich reduziert werden.

### Beispiel 7

Eine männliche Testperson mit Dünndarmresektion (60% des Dünndarms entfernt), beschwerdefrei ab einer Aufteilung der täglichen Nahrungsmenge auf 6 Hauptmahlzeiten, wird behandelt mit den beschichteten Kapseln analog Beispiel 5 in einer Dosis von jeweils einer Kapsel zu drei Hauptmahlzeiten pro Tag. Ab dem dritten Tag wird die gleiche Nahrungsmenge auf 5 Hauptmahlzeiten umgestellt, der Patient bleibt Beschwerdefrei. Ab dem fünften Tag wird die gleiche Nahrungsmenge auf 3 Hauptmahlzeiten und 2 Zwischenmahlzeiten umgestellt. Der Stuhl wird weicher und fettig glänzend, jedoch nicht als unangenehm empfunden. Blähungsbeschwerden, unter denen der Patient bei entsprechend großen Mahlzeiten ohne Behandlung litt, treten nicht auf.

### Beispiel 8

Aufgrund fehlender Untersuchungen über die Absorption von Schwermetallen durch die Dickdarmschleimhaut von Säuglingen wurde kein Behandlungsversuch zur Linderung von Dreimonatskollken durchgeführt. Da die Dreimonatskoliken durch Blähungen verursacht werden, die entstehen, weil der noch nicht vollständig ausgereifte Säuglingsdünndarm die Nährstoffe noch nicht vollständig absorbieren kann, und diese somit durch Dickdarmbakterien zu Gasen verstoffwechseit werden, ist jedoch davon auszugehen, dass auch Dreimonatskoliken bei Säuglingen auf die Behandlung mit der erfindungsgemäßen Zusammensetzung ansprechen. Es wird vorgeschlagen, nach entsprechender Abklärung der Risiken, z.B. Messung der Blutspiegel der entsprechenden Stoffe nach Applikation in den Dickdarm, beginnend mit ungefährlichen Mineralstoffmengen, Säuglinge mit Dreimonatskoliken folgendermaßen zu behandeln: Da für die Behandlung von Säuglingen beschichtete Tabletten ungeeignet sind, wird ein Wirkstoffgemisch aus Natriumselenit und Natriumwolframat durch dem Fachmann bekannte Verfahren mit entsprechenden Hilfsstoffen zu einem Mikrogranulat verarbeitet, welches mit einem Polymer beschichtet wird, das sich beim Erreichen des im Dickdarm des Säuglings vorherrschenden PH-Wertes auflöst. Dazu stehen z.B. diverse Eudragit-Formulierungen zur Verfügung. Die Aufbringung der dünndarmresistenten Schicht kann z.B. im Wirbeistromverfahren erfolgen.
Das entsprechend beschichtete Mikrogranulat wird mit Wasser, evtl. unter Zusatz von entsprechenden Säuglingsnahrungspulvem oder mit Muttermilch zu einer Suspension aufgeschüttelt und zur Still- oder Flaschenmahlzeit gegeben,

### Beispiel 9

Ein 25 Jahre Alter Mann, der nach einer Antibiotikabehandlung ein Reizdarmsyndrom entwickelt hatte, und alle zwei bis 3 Tage über starke Blähungsbeschwerden klagt, wird behandelt mit den beschichteten Kapseln analog Beispiel 5 in einer Dosis von jeweils einer Kapsel zu jeder der drei Hauptmahlzeiten pro Tag. Während der gesamten Testphase von 8 Tagen besteht Beschwerdefreiheit.

### Beispiel 10

Eine 35-jährige Frau mit einem durch Morbus Crohn geschädigten Darm klagt seit dem letzten Krankheitsschub über erhebliche Flatulenz (40-50 bewusst wahrgenommene Flatus während des Tages). Sie wird behandelt mit den beschichteten Kapseln analog Beispiel 5 in einer Rosts von jeweils einer Kapsel zu jeder der drei Hauptmahlzeiten pro Tag. Nach 5 Tagen Behandlung lässt die Flatulenz deutlich nach (bis auf 15 bewusst wahrgenommene Flatus während des siebten Tages)

### Beispiel 11

Eine 42 jährige Mann, der an einer Gluten-Unverträglichkeit leidet, und an einer dadurch bedingten Schädigung der Dünndarmschleimhaut und schmerzhaften Blähungen, wird behandelt mit den beschichteten Kapseln analog Beispiel 5 in einer Dosis von jeweils einer Kapsel zu jeder der drei Hauptmahlzeiten pro Tag. Nach 5 Tagen Behandlung kann eine deutliche Verminderung der Blähungen festgestellt werden.

### Beispiel 12

Eine 26-jährige Frau mit einer Histaminintoleranz, die nach dem Verzehr von histaminreichen Nahrungsmitteln unter starken Blähungen leidet, wird behandelt mit den beschichteten Kapseln analog Beispiel 5 in einer Dosis von jeweils einer Kapsel zu jeder der drei Hauptmahlzeiten pro Tag. Nach 4 Tagen Behandlung kann eine Verminderung der Blähungen festgestellt werden, nach 10 Tagen Behandlung ist die Verminderung der Blähungen deutlich feststellbar.

### Beispiel 13

Analog zu Beispiel 5 werden Kapseln hergestellt, mit dem Unterschied, dass beim Abfüllen der Wirkstoffmischung in die Kapsel zusätzlich gefriergetrocknete. lebensfähige Bakterien der Spezies E.coli Nissle 1917 in einer Menge von 8*10⁹ koloniebildenden Einheiten (KBE) eingefüllt werden.

Einer 21-Jährige Frau, die aufgrund einer Lactoseintoleranz nach dem Verzehr von Milchprodukten unter Durchfällen leidet, wird zu jeder Hauptmahlzeit (3 mal täglich) eine der beschriebenen Kapseln verabreicht. Nach einer Behandlungsdauer von einer Woche erhöht sich durch die Behandlung die Lactosetoleranz der Patientin von 2g pro Mahlzeit auf 4g.

Daraufhin wird die Medikation verdoppelt (3 mal 2 Kapseln täglich) und die Toleranzgrenze steigt auf 8g Lactose pro Mahlzeit.

### Beispiel 14

Analog zu Beispiel 4 werden Kapseln hergestellt, die jedoch im Gegensatz zu Beispiel 4 nicht mit Eudragit FS 30 D dünndarmresistent, sondern mit Eudragit L 30 D-55 magensaftresistent geschichtet werden.
Die Verwendung erfolgt analog Beispiel 1, 2, 5 bis 7 oder 9 bis 12.

### Beispiele 15

Ein 36-jähriger Mann, der nach Verzehr von ballaststoffreichen Mahlzeiten unter starkem Meteorismus leidet, wird behandelt mit dreimal täglich 1,5mg Natriumwolframat, welches in Wasser gelöst mit der Trinkflüssigkeit zu den Hauptmahlzeiten verabreicht wird. Nach einer Woche Behandlung lässt der Meteorismus deutlich nach.

### Beispiel 16

Ein 36-jähriger Mann, der nach Verzehr von ballaststoffreichen Mahlzeiten unter starkem Meteorismus leidet, wird behandelt mit dreimal täglich 1,5mg Natriumwolframat, welches unter Zugabe von 100mg Phytinsäure in 100ml Wasser gelöst und 30 Minuten ruhen gelassen wird, und mit der Trinkflüssigkeit zu den Hauptmahlzeiten verabreicht wird. Nach 4 Tagen Behandlung lässt der Meteorismus deutlich nach.

## Patentansprüche

1. Zusammensetzung, umfassend Wolfram und/oder eine Wolframverbindung zur Verabreichung an ein Säugetier, zur Vermeidung oder Reduzierung von Gasbildung im Dickdarm und dadurch bedingter Blähungen oder Meteorismus.

2. Zusammensetzung zur Verwendung nach Anspruch 1, **dadurch gekennzeichnet, dass** sie galenisch oder chemisch so ausgeführt ist, dass Wolfram und/oder eine Wolframverbindung im Dickdarm des Säugetiers freigesetzt werden, wobei die chemische Ausführung vorzugsweise durch Bindungen des Wolframs oder der Wolframverbindungen an chemische Verbindungen, welche im Dünndarm des Säugetiers nicht spaltbar oder resorbierbar sind, vorzugsweise Phytinsäure, Oxalsäure oder Tannine oder Gerbsäuren, realisiert ist.

3. Zusammensetzung zur Verwendung nach einem der Ansprüche 1 oder 2, **dadurch gekennzeichnet, dass** sie graduell über einen Zeitraum von wenigstens 3 Stunden, vorzugsweise 6 Stunden, besonders bevorzugt 12 Stunden im Dickdarm freigesetzt wird.

4. Zusammensetzung zur Verwendung nach einem der Ansprüche 1 oder 2, dadurch gekenntzeichnet, dass die galenische Ausführung so ausgeführt ist, dass Wolfram und/oder eine Wolframverbindung nach der Magenpassage kontinuierlich über einen Zeitraum von mindestens drei Stunden, bevorzugt bis zu 48 Stunden, freigesetzt werden.

5. Zusammensetzung zur Verwendung nach einem der Ansprüche 1 bis 4, **dadurch gekennzeichnet, dass** sie ferner einen oder mehrere acetogene Bakterienstämme, vorzugsweise ausgewählt aus der Gruppe bestehend aus: Ruminococcus, Eubacterium, Clostridium, enthält.

6. Zusammensetzung zur Verwendung nach einem der Ansprüche 1 bis 5, **dadurch gekennzeichnet, dass** sie ferner einen oder mehrere butyrogene Bakterienstämme, vorzugsweise ausgewählt aus der Gruppe bestehend aus: Fusobacterium, Faecalibacterium, Eubacterium, Clostridium enthält.

7. Zusammensetzung zur Verwendung nach aus einem der Ansprüche 1 bis 6, **dadurch gekennzeichnet, dass** sie ferner Enterobaktarien, vorzugsweise der Gattung Escherichia coli, wahlweise in einer separaten oder dergleiche galenischen Aufbereitung, enthält, wobei die Zusammensetzung stattdessen und/oder zusätzlich zur Behandlung von Durchfall bestimmt ist.

8. Zusammensetzung zur Verwendung nach einem der Ansprüche 1 bis 7, **dadurch gekennzeichnet, dass** die Zusammensetzung stattdessen und/oder zusätzlich zur Unterstützung des Wiederaulbaus oder Regeneration der Darmflora mit acetogenen Bakterien im Anschluss an eine Antibiotikatherapie, Hydro-Colon-Therapie und Darmlavagen oder gastrointestinale Infektionen bestimmt ist.

9. Zusammensetzung zur Verwendung nach einem der Ansprüche 1 bis 8, **dadurch gekennzeichnet, dass** sie zusätzlich Vanadium, Nickel, Eisen. Natrium, Kallum und/oder Vitamine, insbesondere Vitamin A, Vitamin B, Vitamin C, Vitamin D, Vitamin E, Vitamin K enthält.

10. Zusammensetzung zur Verwendung nach einem der Ansprüche 1 bis 9, **dadurch gekennzeichnet, dass** sie zusätzlich Selen und/oder Molybdän enthält.

11. Zusammensetzung zur Verwendung nach einem der Ansprüche 1 bis 10, **dadurch gekennzeichnet, dass** sie zusätzlich eine oder mehrere, vorzugsweise vom Dünndarm des Säugetiers schlecht absorbierbare, Stickstoffverbindungen und/oder Kohlenhydratverbindungen enthält.

12. Zusammensetzung zur Verwendung nach einem der Ansprüche 1 bis 11, **dadurch gekennzeichnet, dass** die Blähungen oder der Meteorismus durch mindestens eine der folgenden gastrointestinalen Störungen verursacht werden/sind: Fructosemalabsorption Fructoseintoleranz, Lactoseintoleranz, Irritated Bowel Syndrom, Disaccharasemangel, Trehafasemangel, Kurzdarmsyndrom, Reizdarmsyndrom, Morbus Crohn, Dreimonatskoliken, exokrine Pankreasinsuffizienz, Gallensäuremangel, Magensäuremangel, antibiotikainduzierte Dysbiose der Darmflora, Mangel an acetogenen Bakterien, Mangel an reduktiv acetogener Stoffwechselleistung der Darmflora, Zölliakie, Sprue, Gluten-Unverträglichkeit, Histaminintoleranz.

13. Zusammensetzung zur Verwendung nach einem der Ansprüche 1 bis 12, **dadurch gekennzeichnet, dass** die Verabreichung oral oder rektal erfolgt.

14. Zusammensetzung zur Verwendung nach einem der Ansprüche 1 bis 13, **dadurch gekennzeichnet, dass** es sich bei dem Säugetier um einen Menschen handelt.

15. Verwendung einer Zusammensetzung nach einem der Ansprüche 1 bis 13 zur Herstellung eines Arzneimittels zur Vermeidung oder Reduzierung von Gasbildung im Dickdarm und dadurch bedingter Blähungen oder Meteorismus.

## Claims

1. A composition comprising tungsten and/or a tungsten compound for the administration to a mammal for the prevention or reduction of gas formation in the colon and thus induced bloating or meteorism.

2. A composition for the use according to claim 1, **characterized in that** it is carried out chemically or galenically in a way that tungsten and/or a tungsten compound is released in the colon, where the chemical implementation is realised preferably by bonding of the tungsten and/or a tungsten compound to chemical compounds which are not cleavable or absorbable in the small intestine of the mammal, preferably phytic acid, oxalic acid or tannins or tannic acids.

3. A composition for the use according to one of the claims 1 or 2, **characterized in that** it is gradually released in the large intestine over a period of at least three hours, preferably 6 hours, particularly preferred 12 hours.

4. A composition for the use according to one of the claims 1 or 2, **characterized in that** the galenic implementation is designed in a way that tungsten and/or a tungsten compound are continuously released over a period of at least three hours, preferably up to 48 hours, after passage of the stomach.

5. A composition for the use according to one of the claims 1 to 4, **characterized in that** it further contains one or more strains of acetogenic bacteria, preferably selected from the group consisting of: Ruminococcus, Eubacterium and Clostridium.

6. A composition for the use according to one of the claims 1 to 5, **characterized in that** it further contains one or more strains of butyrogenic bacteria, preferably selected from the group consisting of: Fusobacterium, Faecalibacterium, Eubacterium and Clostridium.

7. A composition for the use according to one of the claims 1 to 6, **characterized in that** it further contains enterobacteria, preferably of the species Escherichia coli, either in a separate or the same galenic preparation, whereas the composition is instead and / or additionally determined for the treatment of diarrhea.

8. A composition for the use according to one of the claims 1 to 7, **characterized in that** it is instead and/or additionally determined for the support of the reconstruction or regeneration of the intestinal flora with acetogenic bacteria following an antibiotic therapy, hydro-colon-therapy and intestinal lavages or gastrointestinal infections.

9. A composition for the use according to one of the claims 1 to 8, **characterized in that** it additionally contains vanadium, nickel, iron, sodium, potassium, and / or vitamins, especially Vitamin A, Vitamin B, Vitamin C, Vitamin D, Vitamin E, Vitamin K.

10. A composition for the use according to one of the claims 1 to 9, **characterized in that** it additionally contains selenium and / or molybdenum.

11. A composition for the use according to one of the claims 1 to 10, **characterized in that** it additionally contains one or more nitrogen containing compounds and / or carbohydrate compounds, preferably ones that are poorly absorbable by the small intestine of the mammal.

12. A composition for the use according to one of the claims 1 to 11, **characterized in that** the bloating or the meteorism are caused by at least one of the following gastrointestinal disorders: Fructose malabsorption, fructose intolerance, lactose intolerance, irritated bowel syndrome, lack of disaccharase, lack of trehalase, short bowel syndrome, irritable bowel syndrome, crohn's disease, three month colic, exocrine pancreatic insufficiency, bile acid deficiency, gastric acid deficiency, dysbiosis of the intestinal flora caused by antibiotics, lack of acetogenic bacteria, lack of reductive acetogenic metabolic function of the intestinal flora, celiac disease, sprue, gluten intolerance, histamine intolerance.

13. A composition for the use according to one of the claims 1 to 12, **characterized in that** the administration takes place oral or rectal.

14. A composition for the use according to one of the claims 1 to 13, **characterized in that** the mammal is a human.

15. Use of a composition according to one of the claims 1 to 13 for the manufacturing of a medicament for the prevention or reduction of gas formation in the colon and thus induced bloating or meteorism.

## Revendications

1. Composition comprenant du tungstène et/ou un composé de tungstène destinée à une administration à un mammifère, pour éviter ou réduire la formation de gaz dans le gros intestin et de ce fait les ballonnements ou le météorisme.

2. Composition destinée à une utilisation selon la revendication 1, **caractérisée en ce qu'**elle est exécutée sur le plan galénique ou chimique de façon à ce que le tungstène et/ou le composé de tungstène soit libéré dans le gros intestin du mammifère, l'exécution chimique étant réalisée de préférence par des liaisons du tungstène ou des composés de tungstène à des composés chimiques qui ne peuvent être clivés ou résorbés dans l'intestin grêle du mammifère, de préférence à l'acide phytique, à l'acide oxalique ou à des tannins ou des acides tanniques.

3. Composition destinée à une utilisation selon l'une des revendications 1 ou 2, **caractérisée en ce qu'**elle est libérée graduellement dans le gros intestin sur une durée au moins égale à 3 heures, de préférence à 6 heures, de façon particulièrement préférée à 12 heures.

4. Composition destinée à une utilisation selon l'une des revendications 1 ou 2, **caractérisée en ce qu'**elle est exécutée sur le plan galénique de façon telle que le tungstène et/ou un composé de tungstène est libéré après le tractus gastrique de façon continue sur une durée au moins égale à trois heures, de préférence allant jusqu'à 48 heures.

5. Composition destinée à une utilisation selon l'une des revendications 1 à 4, **caractérisée en ce qu'**elle contient en outre une ou plusieurs souches de bactéries acétogènes, de préférence sélectionnées dans le groupe composé de : Ruminococcus, Eubacterium, Clostridium.

6. Composition destinée à une utilisation selon l'une des revendications 1 à 5, **caractérisée en ce qu'**elle contient en outre une ou plusieurs souches de bactéries butyrogènes, de préférence sélectionnées dans le groupe composé de : Fusobacterium, Faecalibacterium, Eubacterium, Clostridium.

7. Composition destinée à une utilisation selon l'une des revendications 1 à 6, **caractérisée en ce qu'**elle contient en outre des entérobactéries, de préférence de l'espèce Escherichia coli, éventuellement dans une préparation galénique séparée ou dans la même préparation galénique, la composition étant par contre et/ou en outre destinée au traitement de la diarrhée.

8. Composition destinée à une utilisation selon l'une des revendications 1 à 7, **caractérisée en ce qu'**elle est au lieu de cela et/ou en outre destinée à soutenir la restauration ou la régénération de la flore intestinale par des bactéries acétogènes suite à une antibiothérapie, une hydrothérapie du côlon et des lavages intestinaux ou des infections gastro-intestinales.

9. Composition destinée à une utilisation selon l'une des revendications 1 à 8, **caractérisée en ce qu'**elle contient en outre du vanadium, du nickel, du fer, du sodium, du potassium et/ou des vitamines, en particulier de la vitamine A, de la vitamine B, de la vitamine C, de la vitamine D, de la vitamine E, de la vitamine K.

10. Composition destinée à une utilisation selon l'une des revendications 1 à 9, **caractérisée en ce qu'**elle contient en outre du sélénium et/ou du molybdène.

11. Composition destinée à une utilisation selon l'une des revendications 1 à 10, **caractérisée en ce qu'**elle contient en outre un ou plusieurs composés azotés et/ou composés d'hydrates de carbone, de préférence difficilement absorbables par l'intestin grêle du mammifère.

12. Composition destinée à une utilisation selon l'une des revendications 1 à 11, **caractérisée en ce que** les ballonnements et/ou le météorisme est/sont provoqué(s) par au moins l'un des dérèglements gastro-intestinaux suivants : mauvaise absorption du fructose, intolérance au fructose, intolérance au lactose, syndrome de l'intestin irrité, carence en disaccharase, carence en tréhalase, syndrome de l'intestin court, syndrome du côlon irritable, maladie de Crohn, coliques des trois mois, insuffisance pancréatique exocrine, carence en acide biliaire, carence en acide gastrique, dysbiose de la flore intestinale induite par des antibiotiques, carence en bactéries acétogènes, carence en performance métabolique acétogène réductive de la flore intestinale, maladie coeliaque, diarrhée tropicale, intolérance au gluten, intolérance à l'histamine.

13. Composition destinée à une utilisation selon l'une des revendications 1 à 12, **caractérisée en ce que** l'administration se fait par voie orale ou rectale.

14. Composition destinée à une utilisation selon l'une des revendications 1 à 13, **caractérisée en ce qu'**il s'agit concernant le mammifère d'un humain.

15. Utilisation d'une composition selon l'une des revendications 1 à 13 pour fabriquer un médicament destiné à éviter ou réduire la formation de gaz dans le gros intestin et les ballonnements ou le météorisme qu'ils impliquent.
